# EUROPEAN PATENT SPECIFICATION

(11) **EP 3 791 887 B1**
(45) Date of publication and mention of the grant of the patent: **23.10.2024**
(21) Application number: 19799696.0
(22) Date of filing: 07.05.2019
(51) Int. Cl.: A01K 67/0276, C12N 9/02, C12N 9/10, C12N 5/078

(54) **METHOD OF PREPARING A BLOOD PRODUCT FROM GENE KNOCKOUT PIG**
VERFAHREN ZUR HERSTELLUNG EINES BLUTPRODUKTS AUS EINEM GEN-KNOCKOUT-SCHWEIN
PROCÉDÉ DE PRÉPARATION D'UN PRODUIT SANGUIN À PARTIR D'UN PORC KNOCK-OUT

(30) Priority: 07.05.2018 CN 201810426018
(43) Date of publication of application: 17.03.2021
(73) Proprietor: GCreatene (Suzhou) Biotechnology Co., Ltd., Suzhou, Jiangsu 215216 (CN)
(72) Inventor: DAI, Yifan, Suzhou, Jiangsu 215126 (CN); YANG, Haiyuan, Suzhou, Jiangsu 215126 (CN); WANG, Ying, Suzhou, Jiangsu 215126 (CN)
(74) Representative: Laine IP Oy
(86) International application number: PCT/CN2019/085773
(87) International publication number: WO 2019/214591

(56) References cited:
- WO-A1-2008/144940
- CN-A- 107 106 607
- CN-A- 108 220 294
- CN-A- 108 588 123
- US-A1- 2017 311 579
- CEM KUSCU ET AL: "Genome-wide analysis reveals characteristics of off-target sites bound by the Cas9 endonuclease", NATURE BIOTECHNOLOGY, vol. 32, no. 7, 18 May 2014 (2014-05-18), New York, pages 677 - 683, XP055382577, ISSN: 1087-0156, DOI: 10.1038/nbt.2916
- Y. LIN ET AL: "CRISPR/Cas9 systems have off-target activity with insertions or deletions between target DNA and guide RNA sequences", NUCLEIC ACIDS RESEARCH, vol. 42, no. 11, 17 June 2014 (2014-06-17), pages 7473 - 7485, XP055186074, ISSN: 0305-1048, DOI: 10.1093/nar/gku402
- JOSE L. ESTRADA ET AL: "Evaluation of human and non-human primate antibody binding to pig cells lackingGGTA1/CMAH/b4GalNT2 genes", XENOTRANSPLANTATION, vol. 22, no. 3, 1 March 2015 (2015-03-01), US, pages 194 - 202, XP055770346, ISSN: 0908-665X, DOI: 10.1111/xen.12161
- GUERARD W. BYRNE ET AL: "Cloning and expression of porcine beta1,4 N-acetylgalactosaminyl transferase encoding a new xenoreactive antigen", XENOTRANSPLANTATION, vol. 21, no. 6, 1 September 2014 (2014-09-01), US, pages 543 - 554, XP055481364, ISSN: 0908-665X, DOI: 10.1111/xen.12124

## Description

### TECHNICAL FIELD

The application belongs to the field of genetic engineering, and specifically relates to a method of preapring blood product derived from a gene knockout pig by using a CRISPR/Cas9 vector combination.

### BACKGROUND

With the continuous progress of modern medicine, blood transfusion has been widely used in clinical practice, but it is also facing more and more problems, including serious risk of blood transfusion caused by high incidence of infectious diseases such as Acquired Immune Deficiency Syndrome, Hepatitis B, Hepatitis C, etc. Blood demand is increasing and the amount of blood donation is relatively reduced, and blood resources are becoming increasingly scarce. Animal red blood cells (RBCs) can be used to develop alternatives to human RBCs for blood transfusion. Porcine red blood cells (pRBCs) have been widely used in the development of heterogeneous blood transfusion red blood cells, and there are a lot of similarities between pRBCs and human RBCs. Meanwhile, when raised under conditions free from specific pathogens and biosafety, pRBCs do not carry human pathogenic microorganisms. The pRBCs do not express MHC antigens, i.e., swine leukocyte antigens (SLA), therefore the immunogenicity is reduced. There are no nuclei in pRBCs, so it is impossible for pRBCs to carry porcine endogenous retrovirus.

However, the direct use of wild-type pRBCs in clinical blood transfusion may cause many problems. The infusion of wild-type pRBCs into a primate may result in a consistent hyperacute rejection. For example, since wild-type pRBCs express Gal and non-Gal antigens in human with natural hemolytic antibodies, so the lysis of blood cells can be directly caused by antibody-antigen binding and/or complement activation.

Therefore, it is urgently needed at present to acquire modified pRBCs which can be directly used for human blood transfusion.

### SUMMARY OF THE INVENTION

The application provides for a method of preparing a blood product according to claim 1. The blood product has at least one of the following properties: 1) the binding to immunoglobulin in human serum is significantly reduced; 2) having an significant effect on overcoming hyperacute rejection; 3) effectively solving the problem of blood shortage in clinical practice; 4) providing valuable material resources for clinical blood transfusion; 5) effectively knocking out the GGTA1 gene in pigs; 6) effectively knocking out the CMAH gene in pigs; 7) effectively knocking out the β4GalNT2 gene in pigs; 8) being suitable for mass production; 9) effective quality control; and/or 10) safe and reliable, without carrying pathogenic microorganisms and/or viruses.

It is surprisingly found in the application that, by knocking out appropriate exons in the genes to be knocked out, for example, by designing a suitable targeting SgRNA sequence against specific exons in the genes to be knocked out, the knockout efficiency of the genes to be knocked out (for example, comprising the GGTA1 gene, the CMAH gene and/or the β4GalNT2 gene) can be significantly improved by means of a CRISPR/Cas9 vector combination. A blood product derived from the gene knockout pig is then obtained, thus effectively reducing the hyperacute rejection caused by xenotransplantation, allowing it to be an alternative for human blood and play a variety of experimental and clinical functions.

A blood product may derive from a gene knockout pig, a GGTA1 gene, a CMAH gene and a β4GalNT2 gene of the gene knockout pig are knocked out, wherein one or more nucleotides in the β4GalNT2 gene encoding one or more amino acids in exon 8 are deleted such that the β4GalNT2 gene is knocked out.

One or more nucleotides in the GGTA1 gene encoding one or more amino acids in exon 3 are deleted such that the GGTA1 gene is knocked out.

One or more nucleotides in the CMAH gene encoding one or more amino acids in exon 6 are deleted such that the CMAH gene is knocked out.

The gene knockout pig is prepared by using a CRISPR/Cas9 vector combination.

According to the invention , the exon 3 of the GGTA1 gene, the exon 6 of the CMAH gene and the exon 8 of the β4GalNT2 gene serve as the parts targeted by CRISPR/Cas9.

The CRISPR/Cas9 vector combination comprises a GGTA1-CRISPR/Cas9 vector, a CMAH-CRISPR/Cas9 vector and a β4GalNT2-CRISPR/Cas9 vector, the GGTA1-CRISPR/Cas9 vector contains the SgRNA nucleotide sequence specifically targeting the GGTA1 gene of SEQ ID No: 1, the CMAH-CRISPR/Cas9 vector contains the SgRNA nucleotide sequence specifically targeting the CMAH gene of SEQ ID No: 2, and the β4GalNT2-CRISPR/Cas9 vector contains the SgRNA nucleotide sequence specifically targeting the β4GalNT2 gene of SEQ ID No: 3.

In some embodiments, the GGTA1-CRISPR/Cas9 vector comprises the nucleotide sequence of SEQ ID No: 4, the CMAH-CRISPR/Cas9 vector comprises the nucleotide sequence of SEQ ID No: 5, and the β4GalNT2-CRISPR/Cas9 vector comprises the nucleotide sequence of SEQ ID No: 6.

In some embodiments, the blood product comprises red blood cells of the gene knockout pig. In some embodiments, the blood product comprises peripheral blood mononuclear cells (PBMC) of the gene knockout pig.

In some embodiments, the red blood cells have a reduced aGal antigen level, a reduced Neu5Gc antigen level and a reduced Sd^{a}-like antigen level.

In some embodiments, the PBMC have a reduced aGal antigen level, a reduced Neu5Gc antigen level and a reduced Sd^{a}-like antigen level.

In some embodiments, the binding level of the red blood cells of the gene knockout pig to human immunoglobulin is reduced compared to red blood cells derived from a wild-type pig. In some embodiments, the binding level of the PBMC of the gene knockout pig to human immunoglobulin is reduced compared to PBMC derived from a wild-type pig.

In some embodiments, the red blood cells of the gene knockout pig have a comparable level of binding to human immunoglobulin compared to human-derived red blood cells. In some embodiments, the PBMC of the gene knockout pig have a comparable level of binding to human immunoglobulin compared to human-derived PBMC.

In some embodiments, the human immunoglobulin comprises human IgG and/or human IgM.

In some embodiments, the agglutination reaction of the red blood cells of the gene knockout pig to human serum is reduced compared to red blood cells derived from a wild-type pig. In some embodiments, the agglutination reaction is caused by an IgM antibody against a blood group antigen and/or an IgG antibody against a blood group antigen.

In some embodiments, the likelihood of a hemolytic transfusion reaction occurring after the red blood cells of the gene knockout pig are introduced into a human body is reduced compared to red blood cells derived from a wild-type pig.

The blood product may be used in the preparation of blood products for infusion into human.

The blood products for infusion into human do not substantially cause and/or are capable of ameliorating hyperacute rejection.

### DETAILED DESCRIPTION

In this application, the term "gene knockout" generally refers to a genetic engineering means for silencing a gene and/or failing to express its encoded protein. For example, the gene knockout can use the CRISPR/Cas system.

In this application, the term "GGTA1 gene" generally refers to a gene encoding α-1,3-galactosyl transferase (GGTA1). In this application, the accession number of the GGTA1 gene of the pig *(Sus scrofa*) in Ensemble is ENSSSCG00000005518. The accession number of the GGTA1 pseudogene of the pig in GenBank is 396733.

In this application, the term "CMAH gene" generally refers to a gene encoding cytidine monophospho-N-acetylneuraminic acid hydroxylase (CMAH). In this application, the accession number of the CMAH gene of the pig *(Sus scrofa*) in Ensemble is ENSSSCG00000001099. The accession number of the CMAH gene of the pig in GenBank is 396918.

In this application, the term "β4GalNT2 gene" generally refers to a gene encoding β-1,4-N-acetyl galactosaminyl transferase 2 (β4GalNT2). In this application, the accession number of the β4GalNT2 gene of the pig (*Sus scrofa*) in Ensemble is ENSSSCG00000040942. The accession number of the β4GalNT2 gene of the pig in GenBank is 100621328. The β4GalNT2 gene of the pig and its products may be important non-Gal antigens that cause xenotransplantation rejection.

In this application, the term "exon" generally refers to a part of an eukaryotic gene. In this application, the exon may be a coding gene which can be still preserved after splicing and can be expressed as a protein during the protein biosynthesis. The exon can also be known as an expression sequence. A linearly expressed eukaryotic gene may have multiple exons. For example, the exon may be blocked with an intron. In this application, exon X (X is a positive integer) may represent the X^{th} exon of the gene.

In this application, the term "blood product" generally refers to blood and products prepared from blood. For example, the blood product may comprise blood, red blood cells and their constituent products, platelets and their constituent products, plasma, plasma protein products and/or coagulation factor products.

In this application, the term "CRISPR/Cas9 vector combination" generally refers to a combination of vectors comprising CRISPR (i.e., Clustered regularly interspaced short palindromic repeats, CRISPR) and the Cas gene. In this application, the CRISPR/Cas9 vector combination may be utilized to realize the gene knockout (see Deveau et al., 2008; Horvath and Barrangou, 2010).

In this application, the term "SgRNA" generally refers to a single-stranded chimeric antibody RNA (Single guide RNA, sgRNA) in an artificial CRISPR/Cas9 system (see Deltcheva et al., 2011; Bikard and Marraffini, 2013).The SgRNAmay be about 20bp in length. In this application, the SgRNA can bind to a target sequence, and then bind to the Cas9 protein to form a complex.

In this application, the term "red blood cells" generally refers to cells in the blood which deliver oxygen to each tissue, also known as "erythrocyte"or "red blood cells". The primary functional molecules of red blood cells may be hemoglobin, which can bind to oxygen molecules in lung, then release the bound oxygen molecules in tissues. In this application, the red blood cells can also deliver carbon dioxide. In this application, the red blood cells in mammals (for example, pigs) may have no nuclei. The red blood cells may also have no mitochondria.

In this application, the term "peripheral blood mononuclear cells (PBMC)" generally refers to peripheral blood mononuclear cell with round nuclei. PBMC may comprise lymphocytes (for example, T cells, B cells or NK cells) and monocytes. PBMC may be extracted from whole blood of mammals (for example, pigs) (for example, may be obtained by gradient centrifugation).

In this application, the term "αGal antigen" generally refers to enzymes (GT, αGal or α1,3 galactosyl transferase) encoded by genes of α1,3galactosyl transferase (aGal, GGTA, GGT1, GT, αGT, GGTA1 or GGTA-1). The αGal antigen may be an epitope or antigen recognized by human immune system. Removal of αGal antigens from transgenic organ materials cannot eliminate the human immune response caused by the materials.

In this application, the term "Neu5Gc antigen" generally refers to N-glycolylneuraminic acid (Neu5Gc).The Neu5Gc antigen may be a sialic acid generated by the catalysis of the CMAH. In this application, the CMAH can catalyze the conversion of sialic acid N-acetyl neuraminic acid (Neu5Ac) to Neu5Gc. The Neu5Gc antigen may be an epitope or antigen recognized by human immune system.

In this application, the term "Sd^{a}-like antigen" generally refers to a glycosyl transferase of a pig. The Sd^{a}-like antigen may be synthesized by the catalysis of β 1,4N-acetyl galactosaminyl transferase. In this application, the Sd^{a}-like antigen may comprise Sd^{a} and similar glycans associated with blood group or blood tests.

In this application, the term "a wild-type pig" generally refers to any known pig (*Sus scrofa*) without any modifications at genetic and/or protein level (for example, deletion, insertion, substitution and/or modification of one or more nucleotides and/or one or more amino acids). For example, the wild-type pig may be *Sus scrofa domestica,* for example may be Berkshire, Chester White, Duroc, Hampshire, Hereford Pig, Landrace, Poland-China swine, Spotted pig or Yorkshire. For example, the wild-type pig may be a wild boar. In this application, the wild-type pig may be a complete individual pig, or may be organs, tissues, body liquid and/or cells of a pig.

In this application, the term "human immunoglobulin" generally refers to an immunologic substance produced by the human immune system after antigen stimulation. For example, the human immunoglobulin may comprise IgA, IgD, IgE, IgG and IgM subtypes. The human immunoglobulin may be known as an antibody (for example, IgG subtype), its monomer may be a Y-shaped molecule, and the antibody may be composed of 4 polypeptide chains, comprising two identical heavy chains and two identical light chains, wherein the light chains and the heavy chains may comprise variable portions -V regions (also known as variable regions), and constant portions - C regions (also known as constant regions).

In this application, the term "blood group antigen" generally refers to an antigen of blood types A, B, AB and O in the AB antigen standard contained in human red blood cells.

In this application, the term "agglutination reaction" generally refers to a serological reaction caused by the binding of an antigen to an antibody. In this application, the antigen (for example, the red blood cells of the gene knockout pig) may be known as agglutinogen. The antibody (for example, an IgM antibody against a blood group antigen and/or an IgG antibody against a blood group antigen) may be known as agglutinin. The agglutination reaction may be characterized by the appearance of small clumps of agglutination visible to the naked eye.

In this application, the term "hemolytic transfusion reaction" generally refers to immune hemolytic transfusion reaction. For example, when the blood recipient is injected incompatible red blood cells or donor plasma with the presence of alloantibodies, the incompatible red blood cells and/or the donor plasma may result in damages to the red blood cells. For example, the hemolytic transfusion reaction may comprise acute (for example, reaction occurs within 24 hours after the blood transfusion) hemolytic transfusion reaction (AHTR) and delayed (for example, reaction occurs several days or weeks after the blood transfusion) hemolytic transfusion reaction (DHTR). For example, the hemolytic transfusion reaction may comprise intravascular hemolysis and extravascular hemolysis.

In this application, the term "hyperacute rejection" (HAR) generally refers to a failure occurred rapidly (for example, within several minutes after the transplantation) after the transplantation of exogenous organs, tissues and/or cells into a recipient. The HAR may occur in heart and/or kidney. The HAR may be associated with thymic T cells.

In this application, the term "Gal" generally refers to a terminal oligosaccharide generated from α1,3-galactosyl transferase (GGTA1). In mammals (e.g., human), the Gal may be the primary binding antigen for antibodies naturally produced in vivo. In this application, all antibodies or antigen-binding fragments thereof that do not bind to the Gal may be considered to be non-Gal antibodies.

In one aspect, the application provides for a method of preparing a blood product as claimed in claim 1.

For example, one or more (for example, 1, 2, 3, 4, 5, 6, 7, 8, 9, 10 or more) nucleotides in the β4GalNT2 gene encoding one or more (for example, 1, 2, 3, 4, 5, 6, 7, 8, 9, 10 or more) amino acids in exon 8 may be deleted or added such that the β4GalNT2 gene is knocked out and/or does not express functional β4GalNT2 encoded products. In this application, the knockout efficiency of the β4GalNT2 gene may be more than about 40%, for example, may be more than about 41%, more than about 42%, more than about 43%, more than about 44%, more than about 45%, more than about 46%, more than about 47%, more than about 48%, more than about 49% or more than about 50%.

In this application, one or more (for example, 1, 2, 3, 4, 5, 6, 7, 8, 9, 10 or more) nucleotides in the GGTA1 gene encoding one or more (for example, 1, 2, 3, 4, 5, 6, 7, 8, 9, 10 or more) amino acids in exon 3 may be deleted or added such that the GGTA1 gene is knocked out and/or does not express functional GGTA1 encoded products. In this application, the knockout efficiency of the GGTA1 gene may be more than about 55%, for example, may be more than about 56%, more than about 57%, more than about 58%, more than about 59%, more than about 60%, more than about 65%, more than about 70%, more than about 75%, more than about 80% or more than about 85%.

In this application, one or more (for example, 1, 2, 3, 4, 5, 6, 7, 8, 9, 10 or more) nucleotides in the CMAH gene encoding one or more (for example, 1, 2, 3, 4, 5, 6, 7, 8, 9, 10 or more) amino acids in exon 6 may be deleted or added such that the CMAH gene is knocked out and/or does not express functional CMAH encoded products. In this application, the knockout efficiency of the CMAH gene may be more than about 60%, for example, may be more than about 61%, more than about 62%, more than about 63%, more than about 64%, more than about 65%, more than about 70%, more than about 75%, more than about 80% or more than about 85%.

In this application, the gene knockout pig is prepared by using a CRISPR/Cas9 vector combination.

The exon 3 of the GGTA1 gene, the exon 6 of the CMAH gene and the exon 8 of the β4GalNT2 gene serve as the parts targeted by CRISPR/Cas9.

For example, CRISPR target sequence of the GGTA1 gene may be located in exon 3 of the gene, near the initiation codon. Knockout of the GGTA1 gene may further comprise inserting one or more (for example, may insert 1, 2, 3, 4, 5 or more) bases (for example, may insert one base) in exon 3 of the gene. For example, T can be inserted between T and C of exon 3 of the GGTA1 gene.

For example, CRISPR target sequence of the CMAH gene may be located in exon 6 of the gene, near the initiation codon. Knockout of the CMAH gene may further comprise inserting one or more (for example, may insert 1, 2, 3, 4, 5 or more) bases (for example, may insert one base) in exon 3 of the gene. For example, A can be inserted between A and G of exon 6 of the GGTA1 gene.

For example, CRISPR target sequence of the β4GalNT2 gene may be located in exon 8 of the gene. For example, knockout of the β4GalNT2 gene may comprise deleting one or more (for example, may delete 1, 2, 3, 4, 5, 6, 7, 8, 9, 10 or more) bases in exon 8 of the gene. For example, 10 bases may be deleted. For example, bases of ACTCACGAAC may be deleted.

In some instances, CRISPR target sequence of the p4GalNT2 gene may not be located in exon 2 of the gene.

It is surprisingly found in the application that gene knockout in respect of exon 8 of the β4GalNT2 gene can significantly improve the knockout efficiency of the β4GalNT2 gene. Moreover, the knockout efficiency of the β4GalNT2 gene caused by the knockout in respect of exon 8 of the β4GalNT2 gene may be significantly higher than the knockout efficiency of knockout in respect of exon 2 of the β4GalNT2 gene.

In this application, the CRISPR/Cas9 vector combination comprises a GGTA1-CRISPR/Cas9 vector, a CMAH-CRISPR/Cas9 vector and a β4GalNT2-CRISPR/Cas9 vector. The GGTA1-CRISPR/Cas9 vector contains the SgRNA nucleotide sequence specifically targeting the GGTA1 gene of SEQ ID No: 1. The CMAH-CRISPR/Cas9 vector contains the SgRNA nucleotide sequence specifically targeting the CMAH gene of SEQ ID No: 2. And, the β4GalNT2-CRISPR/Cas9 vector contains the SgRNA nucleotide sequence specifically targeting the β4GalNT2 gene of SEQ ID No: 3.

In some instances, the GGTA1-CRISPR/Cas9 vector may comprise the nucleotide sequence of SEQ ID No: 4. In some instances, the CMAH-CRISPR/Cas9 vector may comprise the nucleotide sequence of SEQ ID No: 5. And/or, in some instances, the β4GalNT2-CRISPR/Cas9 vector may comprise the nucleotide sequence of SEQ ID No: 6.

For example, the blood product may comprise red blood cells of the gene knockout pig. Further for example, the blood product may comprise peripheral blood mononuclear cells (PBMC) of the gene knockout pig.

In this application, the red blood cells may have a reduced aGal antigen level, a reduced Neu5Gc antigen level and a reduced Sd^{a}-like antigen level.

In this application, the PBMC may have a reduced aGal antigen level, a reduced Neu5Gc antigen level and a reduced Sd^{a}-like antigen level.

In this application, the GGTA1 gene can encode α1,3 galactosyl transferase. The functional α1,3 galactosyl transferase may catalyze the formation of galactose α1,3-galactose (aGal) residues on glycoproteins. The aGal antigen may be an antigen or an epitope thereof recognized by human immune system.

In this application, the CMAH gene may be responsible to the synthesis of N-glycolyl neuraminic acid (Neu5Gc). For example, the CMAH gene may encode cytidine monophospho-N-acetylneuraminic acid hydroxylase, which can catalyze the conversion of sialic acid N-acetylneuraminic acid to the Neu5Gc antigen. The Neu5Gc antigen may be an antigen or an epitope thereof recognized by human immune system.

In this application, the β4GalNT2 gene can encode β1,4 N-acetylgalactosaminyl transferase 2 glycosyl transferase (β4GalNT2). The functional β4GalNT2 may produce Sd^{a}-like glycans (for example, the Sd^{a}-like antigen). The Sd^{a}-like antigen may be an antigen or an epitope thereof recognized by human immune system.

In this application, for example, the binding level of the red blood cells of the gene knockout pig to human immunoglobulin may be reduced (for example, may be reduced by at least about 5 times, at least about 5.5 times, at least about 6 times, at least about 6.5 times or more) compared to red blood cells derived from a wild-type pig. Further for example, the binding level of the PBMC of the gene knockout pig to human immunoglobulin may be reduced (for example, may be reduced by at least about 50 times, at least about 55 times, at least about 60 times, at least about 65 times, at least about 70 times or more) compared to PBMC derived from a wild-type pig.

In this application, for example, the red blood cells of the gene knockout pig may have a comparable level of binding to human immunoglobulin compared to human-derived red blood cells (for example, may be about 135%~125%, about 125%~115%, about 115%~105%, about 105%~95%, about 95%~85% or about 85%~75% of the binding level of human-derived red blood cells to human immunoglobulin). Further for example, the PBMC of the gene knockout pig may have a comparable level of binding to human immunoglobulin compared to human-derived PBMC (for example, may be about 135%~125%, about 125%~115%, about 115%~105%, about 105%~95%, about 95%~85% or about 85%~75% of the binding level of human-derived PBMC to human immunoglobulin).

In this application, the human immunoglobulin may comprise human IgG and/or human IgM.

In this application, the agglutination reaction of the red blood cells of the gene knockout pig in human serum may be reduced compared to red blood cells derived from a wild-type pig (for example, may be reduced by at least about 1.5 times, at least about 2 times, at least about 2.5 times, at least about 3 times or more). In this application, the agglutination reaction may be caused by an IgM antibody against a blood group antigen and/or an IgG antibody against a blood group antigen.

In this application, the likelihood of a hemolytic transfusion reaction occurring after the red blood cells of the gene knockout pig are introduced into a human body may be reduced compared to red blood cells derived from a wild-type pig (for example, may be reduced by at least about 5 times, at least about 5.5 times, at least about 6 times, at least about 6.5 times or more).

The blood products which may be used for infusion into human may not substantially cause and/or be capable of ameliorating hyperacute rejection. The amelioration aims to make any advance or progress towards the treatment and/or relief of hyperacute rejection. For example, the blood product may be used to ameliorate at least one symptom associated with the hyperacute rejection selected from the group consisting of: thrombotic occlusion, graft vasculature bleeding, neutrophil influx, ischemia, plaque, edema, cyanosis, edema, organ failure, organ dysfunction and/or necrosis, glomerular capillary thrombosis, hemolysis, fever, coagulation, reducedbile production, hypotension, elevated serum transaminase level, elevated alkaline phosphatase level, jaundice, lethargy, acidosis, hyperbilirubinemia, and/or thrombocytopenia.

The blood product may comprise plasma, serum albumin, placental serum albumin, intravenous immunoglobulin, intramuscular immunoglobulin, histamine immunoglobulin, specific immunoglobulin, hepatitis B immunoglobulin, rabies immunoglobulin, tetanus immunoglobulin, blood coagulation factor VIII, prothrombin complex, fibrinogen, antilymphocyte immunoglobulin, antithrombin III, topical lyophilized fibrin adhesive, lyophilized thrombin, and/or S/D-FFP.

To address the immunological rejection present in existing clinical transfusion of xenogeneic red blood cells a CRISPR/Cas9 vector combination may be used in the preparation of blood products for gene knockout pigs.

Technical Solution: The use of the CRISPR/Cas9 vector combination of the application in the preparation of blood products for gene knockout pigs, wherein the gene knockout pigs are pigs with their GGTA1 gene, CMAH gene and β4GalNT2 gene being knocked-out. The CRISPR/Cas9 vector combination comprises a GGTA1-CRISPR/Cas9 vector, a CMAH-CRISPR/Cas9 vector and a β4GalNT2-CRISPR/Cas9 vector; the GGTA1-CRISPR/Cas9 vector contains the SgRNA nucleotide sequence specifically targeting the GGTA1 gene of SEQ ID No: 1, the CMAH-CRISPR/Cas9 vector contains the SgRNA nucleotide sequence specifically targeting the CMAH gene of SEQ ID No: 2, the β4GalNT2-CRISPR/Cas9 vector contains the SgRNA nucleotide sequence specifically targeting the β4GalNT2 gene of SEQ ID No: 3.

For example, the blood products may be red blood cells.

For example, the nucleotide sequence of the GGTA1-CRISPR/Cas9 vector is SEQ ID No: 4; the nucleotide sequence of the CMAH-CRISPR/Cas9 vector is SEQ ID No: 5; the nucleotide sequence of the β4GalNT2-CRISPR/Cas9 vector is SEQ ID No: 6.

Wherein, the CRISPR/Cas9 vector combination can be constructed as follows:
(1) The pX330 plasmid was digested with a BbsI enzyme, and the digested plasmid was separated using an agarose gel, the digestion product was then purified and recovered by a gel extraction kit;
(2) CACC was added at the 5' end of the SgRNA nucleotide sequence to obtain a forward oligonucleotide sequence, and AAAC was added at the 5' end of its complementary strand to obtain a reverse oligonucleotide sequence, thus respectively synthesizing the forward and reverse oligonucleotide sequences, which were then annealed to obtain a double-stranded fragment;
(3) The digestion product obtained in the step (1) was ligated with the double-stranded fragment obtained in the step (2) using a ligase;
(4) The system obtained in the step (3) was treated with a plasmid-safe exonuclease to eliminate the incorrect-ligated plasmid;
(5) The recombinant plasmid obtained in the step (4) was transformed into competent cells for culture;
(6) The recombinant plasmid was extracted from the competent cells cultured in the step (5) and then sequencing to determine the success of vector construction;

When the CRISPR/Cas9 vector is a GGTA1-CRISPR/Cas9 vector, the SgRNA nucleotide sequence in the step (2) is SEQ ID No: 1; when the CRISPR/Cas9 vector is CMAH-CRISPR/Cas9, the SgRNA nucleotide sequence in the step (2) is SEQ ID No: 2; when the CRISPR/Cas9 vector is a β4GalNT2-CRISPR/Cas9 vector, the SgRNA nucleotide sequence in the step (2) is SEQ ID No: 3.

The use of the CRISPR/Cas9 vector combination in the preparation of blood products for gene knockout pigs may comprise the following steps:
(1) The CRISPR/Cas9 vector combination was transformed into porcine fetal fibroblasts;
(2) The fibroblasts obtained in the step (1) were screened for resistance, the resistant fibroblasts were sequenced by PCR amplification to obtain fibroblasts of which the GGTA1 gene, the CMAH gene and the β4GalNT2 gene were knocked out;
(3) The nuclei of fibroblasts obtained in the step (2) were transplanted into denucleated porcine oocytes and cultured to the blastocyst stage;
(4) The blastocysts obtained in the step (3) were transplanted into surrogate pigs for breeding and parturition;
(5) The blood of weaned piglets laboured in the step (4) was drawn through veins and stored in an anticoagulation tube to separate the red blood cells.

The separation step of red blood cells may be as follows: The blood stored in the anticoagulation tube was added into a centrifuge tube, and diluted with a PBS solution. AFicoll-paque separation liquid was added to form a separation system, which was centrifuged to obtain a four-layer solution, including successively, from top to bottom, a plasma layer, a monocyte layer, a Ficoll-paque layer and a red blood cell layer. The top three layers were discarded, and the red blood cells were rinsed with a PBS solution to get a solution of red blood cells. For example, the volume ratio of the blood, the PBS solution and the Ficoll-paque separation liquid in the separation system may be 2:2:3.

For example, the conditions for centrifugation may be: at 19°C, centrifugation at 400 g for 40 min.

In this application, it should be understood that the use of the terms "a/an" and "the" and "at least one" as well as similar indications comprises singular and plural referents. Unless otherwise stated herein or clearly contradicted in the context, when the term "at least one" is used followed by one or more of the listed items (for example, "at least one of A and B"), it should be understood to be selected from one of the listed items (A or B) or any combination of two or more of the listed items (A and B).

In this application, unless otherwise noted, the term " comprise ", "have", "include" as well as "contain" should all be understood as non-exclusive terms (i.e., mean "comprise, but not limited to").

Not wishing to be bound by any theory, the following embodiments are only intended to illustrate the working modes of the device, method and system of the application and are not intended to limit the scope of the invention.

### BRIEF DESCRIPTION OF THE DRAWINGS

The specific characteristics of the invention referred in the application are set forth in the appended claims. The features and advantages of the invention referred in the application can be better understood by referring to the exemplary embodiments described in detail below and the accompanying drawings. A brief description of the drawings is as follows:
Fig. 1 shows SgRNA nucleotide sequences specifically targeting *GGTA1, CMAH* and *β4GalNT2* genes in the CRISPR/Cas9 vector combination;
Fig. 2 shows the plasmid profile of the GGTA1-CRISPR/Cas9 vector;
Fig. 3 shows the plasmid profile of the CMAH-CRISPR/Cas9 vector;
Fig. 4 shows the plasmid profile of the β4GalNT2-CRISPR/Cas9 vector;
Fig. 5 shows the situations of the gene knockout pigs (TKO) of the application at birth and after weaning;
Fig. 6 shows the *GGTAl* gene, *CMAH* gene and *β4GalNT2* gene in the the gene knockout pigs (TKO) of the application being successfully knocked-out;
Figs. 7A-7B show the binding profile of the gene knockout pigs (TKO) of the application to human immunoglobulin;
Fig. 8 shows the antigen flow results of red blood cells of the gene knockout pig (TKO) of the application;
Figs. 9A-9B show the binding results of red blood cells of the gene knockout pig (TKO) of the application to IgM and IgG in human serum;
Fig. 10 shows the results of an agglutination test on red blood cells of the gene knockout pig (TKO) of the application.
Fig. 11 shows the results of an agglutination test on red blood cells of the gene knockout pig (TKO) of the application.
Fig. 12 shows the agglutination titers of red blood cells of the gene knockout pig (TKO) of the application.
Fig. 13 shows the results of an agglutination test on red blood cells of the gene knockout pig (TKO) of the application.
Fig. 14 shows the results of an agglutination test on red blood cells of the gene knockout pig (TKO) of the application.
Fig. 15 shows the results of an MMA test on red blood cells of the gene knockout pig (TKO) of the application.

### EMBODIMENTS

### Embodiment 1 Construction of CRISPR/Cas9 vector

Firstly, based on the DNA sequences of GGTA1/CMAH/β4GalNT2 genes, sgRNA (single guide RNAs) targeting GGTA1, CMAH and β4GalNT2 genes were synthesized, thus constructing a GGTA1-CRISPR/Cas9 vector, a CMAH-CRISPR/Cas9 vector and a β4GalNT2-CRISPR/Cas9 vector respectively, with pX330 as the skeleton plasmid.

### 1.1 Preparation of GGTA1-CRISPR/Cas9 vector

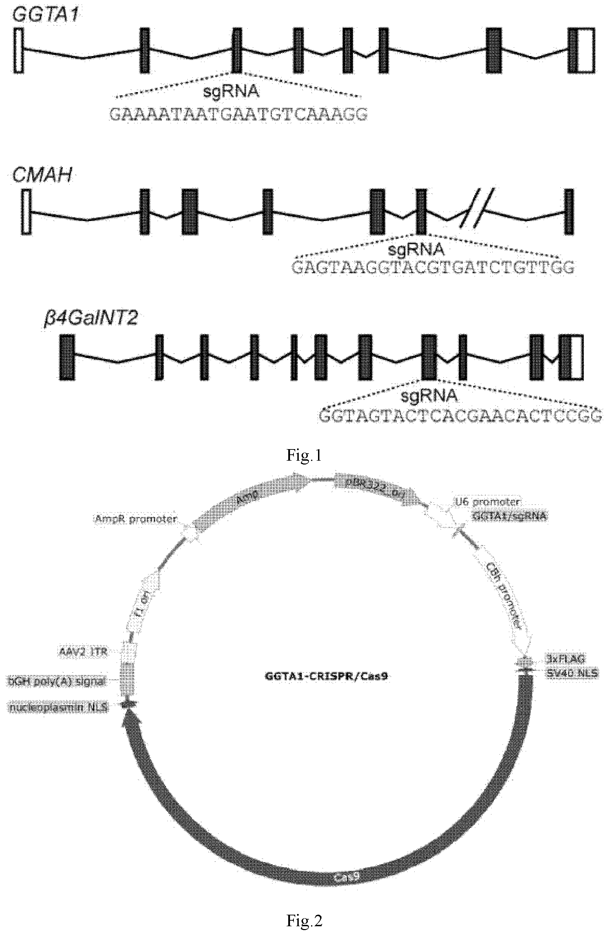

Firstly, based on the porcine GGTA1 gene sequence published in Genbank, the exon 3 of GGTA1 gene was selected as the CRISPR/Cas9 target. According to the design principle of cas9 target, the 5' end was G, and the 3' end was PAM sequence (NGG). The SgRNA sequence was designed to be GAAAATAATGAATGTCAA, as shown in Fig. 1. Its nucleotide sequence is SEQ ID No: 1.

The GGTA1-CRISPR/Cas9 vector was prepared as follows:
Step I. According to the design principle of cas9 target, the 5' end was G, and the 3' end was PAM sequence (NGG), and the target position was found on the GGTA1 gene;
Step II. A pX330 skeleton plasmid (Addgene plasmid 423230) expressing hSpCas9 and gRNA was purchased;
Step III. Synthesis of 5'-end phosphorylated oligonucleotide chain SgRNA sequence: CACC was added at the 5' end of the SgRNA nucleotide sequence to obtain a forward oligonucleotide sequence, and AAAC was added at the 5' end of its complementary strand to obtain a reverse oligonucleotide sequence, thus respectively synthesizing the forward and reverse oligonucleotide sequences:
   5'-CACCGAAAATAATGAATGTCAA-3' (SEQ ID No: 7)
   3'-CTTTTATTACTTACAGTTCAAA-5' (SEQ ID No: 8).

The SgRNA sequence was cloned onto the pX330 skeleton vector, the specific steps of which were as follows:
1. 1 µg pX330 plasmid was digested with a restriction endonuclease BbsI;
2. The digested pX330 plasmid was separated using an agarose gel (an agarose gel at a concentration of 1%, i.e., 1 g agarose gel being added into 100 mL electrophoresis buffer), the digestion product was then purified and recovered by a gel extraction kit (QIAGEN);
3. The forward and reverse oligonucleotide sequences synthesized in step III were annealed as follows:

| | |
|---|---|
| 1 µL | Oligo1 (forward oligonucleotide sequence ) (10 µM) |
| 1 µL | Oligo2 (reverse oligonucleotide sequence ) (10 µM) |
| 8 µL | ddH₂O |
| Total: | 10µL |

At 37°C for 30 min
At 95°C for 5 min, then down to 25°C at a rate of 5°C/min

4. A ligation reaction was initiated following the system below: reaction at room temperature for 10 min

| | |
|---|---|
| pX330 digested with a BbsI enzyme in step 2 | 50 ng |
| 5'-end phosphorylated oligonucleotide after annealing in step 3 (at a volume ratio of 1:250, diluted with sterile water) | 1 µL |
| 2X Rapid ligation buffer (NEB) | 5 µL |
| ddH₂O supplement the system to | 10 µL |
| Subtotal | 10 µL |
| Rapid ligase (NEB) | 1 µL |
| Total | 11 µL |

5. The ligation system was treated with a plasmid-safe exonuclease to eliminate the incorrect-ligated plasmid:

| | |
|---|---|
| Ligation reaction system obtained in step 4 | 11 µL |
| 10X plasmid-safe buffer (NEB) | 1.5 µL |
| 10 mM ATP | 1.5 µL |
| Plasmid-safe exonuclease (NEB) | 1 µL |
| Total | 15 µL |

Reaction at 37°C for 30 min
6. Transformation
(1) 50 µL competent cells (TIANGEN) were placed in an ice bath;
(2) To the centrifuge tube containing the competent cells was added 15 µL the solution with the incorrect-ligated plasmid eliminated obtained in step 5, mixed evenly and left in the ice bath for 30 min;
(3) The competent cells left in the ice bath for 30 min were placed in a water bath at 42°C for 60~90 s, and then transferred into the ice bath quickly to cool the cells for 2 to 3 min;
(4) 900 µL sterile LB medium (free from antibiotics) was added into the centrifuge tube, mixed evenly and placed in a shaking bed at 37°C for culture with shaking at 150 rpm for 45 min;
(5) The centrifuge tube was centrifuged at 12000 rpm in a centrifuge for 5 min. 900 µL supernatant was discarded, and the competent cell precipitates were resuspended with the remaining 100 µL supernatant. The resuspended competent cells were then added onto an LB solid agar medium containing corresponding antibiotics, and coated uniformly with a sterile coating rod; The LB solid agar medium coated with competent cells was inverted in an incubator at 37°C for culture for 12 to 16 h.

7. Mini-extraction of plasmid, sequencing, identification of the successful construction of the target plasmid.

The constructed CRSAPR/Cas9 vector is named as GGTA1-CRISPR/Cas9, its nucleotide sequence is shown in SEQ ID No: 4.

### 1.2. Preparation of CMAH- CRISPR/Cas9 vector

Firstly, based on the porcine CMAH gene sequence published in Genbank, the exon 6 of CMAH gene was selected as the CRISPR/Cas9 target. According to the design principle of cas9 target, the 5' end was G, and the 3' end was PAM sequence (NGG). The SgRNA guide sequence was designed to be GAGTAAGGTACGTGATCTGT, as shown in Fig. 1. Its nucleotide sequence is SEQ ID No: 2.

The CMAH-CRISPR/Cas9 vector was prepared as follows:
Step I. According to the design principle of cas9 target, the 5' end was G, and the 3' end was PAM sequence (NGG), and the target position was found on the CMAH gene;
Step II. A pX330 skeleton plasmid (Addgene plasmid 423230) expressing hSpCas9 and gRNA was purchased;
Step III. Synthesis of 5'-end phosphorylated oligonucleotide chain SgRNA sequence by a company: CACC was added at the 5' end of the SgRNA nucleotide sequence to obtain a forward oligonucleotide sequence, and AAAC was added at the 5' end of its complementary strand to obtain a reverse oligonucleotide sequence, thus respectively synthesizing the forward and reverse oligonucleotide sequences:
   5'-CACCGGAGTAAGGTACGTGATCTGT-3' (SEQ ID No: 9)
   3'-CCTCATTCCATGCACTAGACACAAA-5' (SEQ ID No: 10).

The SgRNA sequence was cloned onto the pX330 skeleton vector, the specific steps of which were as follows:
1. 1 µg pX330 plasmid was digested with a restriction endonuclease BbsI;
2. The digested pX330 plasmid was separated using an agarose gel (an agarose gel at a concentration of 1%, i.e., 1 g agarose gel being added into 100 mL electrophoresis buffer), the digestion product was then purified and recovered by a gel extraction kit (QIAGEN);
3. The forward and reverse oligonucleotide sequences synthesized in step III were annealed as follows:

| | |
|---|---|
| 1 µL | Oligo1 (forward oligonucleotide sequence) (10 µM) |
| 1 µL | Oligo2 (reverse oligonucleotide sequence) (10 µM) |
| 8 µL | ddH₂O |
| Total: | 10µL |

At 37°C for 30 min
At 95°C for 5 min, then down to 25°C at a rate of 5°C/min

4. A ligation reaction was initiated following the system below: reaction at room temperature for 10 min

| | |
|---|---|
| pX330 digested with a BbsI enzyme in step 2 | 50 ng |
| 5'-end phosphorylated oligonucleotide after annealing in step 3 (at a volume ratio of 1:250, diluted with sterile water) | 1 µL |
| 2X Rapid ligation buffer (NEB) | 5 µL |
| ddH₂O supplement the system to | 10 µL |
| Subtotal | 10 µL |
| Rapid ligase (NEB) | 1 µL |
| Total | 11 µL |

5. The ligation system was treated with a plasmid-safe exonuclease to eliminate the incorrect-ligated plasmid:

| | |
|---|---|
| Ligation reaction system obtained in step 4 | 11 µL |
| 10X plasmid-safe buffer (NEB) | 1.5 µL |
| 10 mM ATP | 1.5 µL |
| Plasmid-safe exonuclease (NEB) | 1 µL |
| Total | 15 µL |
| Reaction at 37°C for 30 min | |

6. Transformation
(1) 50 µL competent cells (TIANGEN) were placed in an ice bath;
(2) To the centrifuge tube containing the competent cells was added 15 µL the solution with the incorrect-ligated plasmid eliminated obtained in step 5, mixed evenly and left in the ice bath for 30 min;
(3) The competent cells left in the ice bath for 30 min were placed in a water bath at 42°C for 60~90 s, and then transferred into the ice bath quickly to cool the cells for 2 to 3 min;
(4) 900 µL sterile LB medium (free from antibiotics) was added into the centrifuge tube, mixed evenly and placed in a shaking bed at 37°C for culture with shaking at 150 rpm for 45 min;
(5) The centrifuge tube was centrifuged at 12000 rpm in a centrifuge for 5 min. 900 µL supernatant was discarded, and the competent cell precipitates were resuspended with the remaining 100 µL supernatant. The resuspended competent cells were then added onto an LB solid agar medium containing corresponding antibiotics, and coated uniformly with a sterile coating rod; The LB solid agar medium coated with competent cells was inverted in an incubator at 37°C for culture for 12 to 16 h.

7. Mini-extraction of plasmid, sequencing, identification of the successful construction of the target plasmid.

The constructed CRSAPR/Cas9 vector is named as CMAH-CRISPR/Cas9, its nucleotide sequence is shown in SEQ ID No: 5.

### 1.3. Preparation of β4GalNT2-CRISPR/Cas9 vector

Firstly, based on the porcine β4GalNT2 gene sequence published in Genbank, the exon 8 of β4GalNT2 gene was selected as the CRISPR/Cas9 target. According to the design principle of cas9 target, the 5' end was G, and the 3' end was PAM sequence (NGG). The guide sequence was designed to be GGTAGTACTCACGAACACTC as shown in Fig. 1. Its nucleotide sequence is SEQ ID No: 3.

The β4GalNT2-CRISPR/Cas9 vector was prepared as follows:
Step I. According to the design principle of cas9 target, the 5' end was G, and the 3' end was PAM sequence (NGG), and the target position was found on the β4GalNT2 gene;
Step II. A pX330 skeleton plasmid (Addgene plasmid 423230) expressing hSpCas9 and gRNA was purchased;
Step III. Synthesis of 5'-end phosphorylated oligonucleotide chain SgRNA sequence by a company: CACC was added at the 5' end of the SgRNA nucleotide sequence to obtain a forward oligonucleotide sequence, and AAAC was added at the 5' end of its complementary strand to obtain a reverse oligonucleotide sequence, thus respectively synthesizing the forward and reverse oligonucleotide sequences:
   5'-CACCGGTAGTACTCACGAACACTC-3' (SEQ ID No: 11)
   3'-CCATCATGAGTGCTTGTGAGCAAA-5' (SEQ ID No: 12).

The SgRNA sequence was cloned onto the pX330 skeleton vector, the specific steps of which were as follows:
1. 1 µg pX330 plasmid was digested with a restriction endonuclease BbsI;
2. The digested pX330 plasmid was separated using an agarose gel (an agarose gel at a concentration of 1%, i.e., 1 g agarose gel being added into 100 mL electrophoresis buffer), the digestion product was then purified and recovered by a gel extraction kit (QIAGEN);
3. The forward and reverse oligonucleotide sequences synthesized in step III were annealed as follows:

| | |
|---|---|
| 1 µL | Oligo1 (forward oligonucleotide sequence) (10 µM) |
| 1 µL | Oligo2 (reverse oligonucleotide sequence) (10 µM) |
| 8 µL | ddH₂O |
| Total: | 10 µL |

At 37°C for 30 min
At 95°C for 5 min, then down to 25°C at a rate of 5°C/min

4. A ligation reaction was initiated following the system below: reaction at room temperature for 10 min

| | |
|---|---|
| pX330 digested with a BbsI enzyme in step 2 | 50 ng |
| 5'-end phosphorylated oligonucleotide after annealing in step 3 (1:250 v/v, diluted with sterile water) | 1 µL |
| 2X Rapid ligation buffer (NEB) | 5 µL |
| ddH₂O supplement the system to | 10 µL |
| Subtotal | 10 µL |
| Rapid ligase (NEB) | 1 µL |
| Total | 11 µL |

5. The ligation system was treated with a plasmid-safe exonuclease to eliminate the incorrect-ligated plasmid:

| | |
|---|---|
| Ligation reaction system obtained in step 4 | 11 µL |
| 10X plasmid-safe buffer (NEB) | 1.5 µL |
| 10 mM ATP | 1.5 µL |
| Plasmid-safe exonuclease (NEB) | 1 µL |
| Total | 15 µL |

Reaction at 37°C for 30 min
6. Transformation
(1) 50 µL competent cells (TIANGEN) were placed in an ice bath;
(2) To the centrifuge tube containing the competent cells was added 15 µL the solution with the incorrect-ligated plasmid eliminated obtained in step 5, mixed evenly and left in the ice bath for 30 min;
(3) The competent cells left in the ice bath for 30 min were placed in a water bath at 42°C for 60~90 s, and then transferred into the ice bath quickly to cool the cells for 2 to 3 min;
(4) 900 µL sterile LB medium (free from antibiotics) was added into the centrifuge tube, mixed evenly and placed in a shaking bed at 37°C for culture with shaking at 150 rpm for 45 min;
(5) The centrifuge tube was centrifuged at 12000 rpm in a centrifuge for 5 min. 900 µL supernatant was discarded, and the competent cell precipitates were resuspended with the remaining 100 µL supernatant. The resuspended competent cells were then added onto an LB solid agar medium containing corresponding antibiotics, and coated uniformly with a sterile coating rod; The LB solid agar medium coated with competent cells was inverted in an incubator at 37°C for culture for 12 to 16 h.

7. Mini-extraction of plasmid, sequencing, identification of the successful construction of the target plasmid.

The constructed CRSAPR/Cas9 vector is named as β4GalNT2-CRISPR/Cas9, its nucleotide sequence is SEQ ID No: 6.

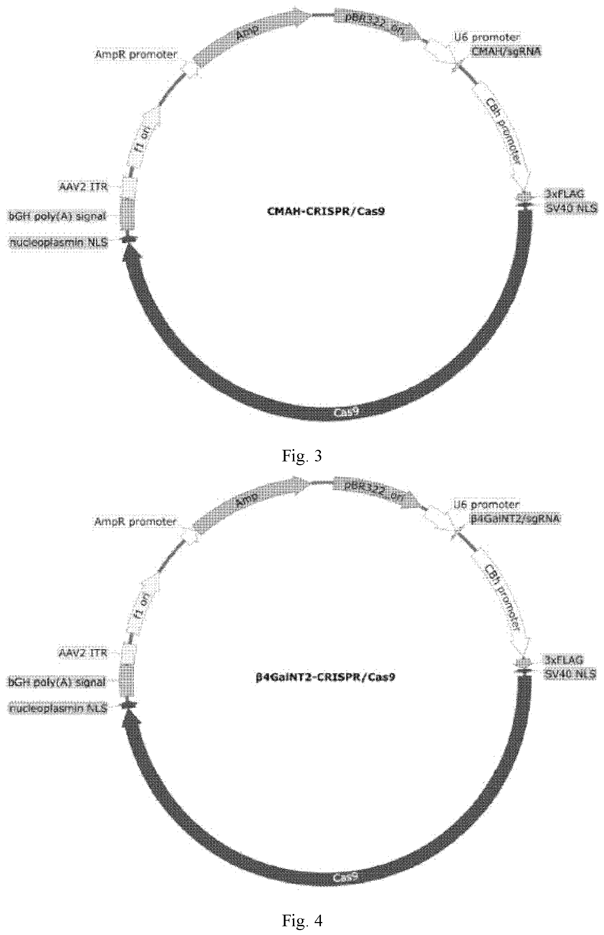

The GGTA1-CRISPR/Cas9 vector, the CMAH-CRISPR/Cas9 vector and the β4GalNT2-CRISPR/Cas9 vector (their profiles can be seen in Figs. 2, 3 and 4 sequentially) widely present in mammals, which express GGTA1/CMAH/β4GalNT2 genes respectively, comprise a U6 promoter, an enhancer of a CMV-chicken-β-actin gene (CMV-chicken-β-actin enhancer), and contain a resistant gene for screening in mammal cells - Neomycin gene and a resistant gene for screening in prokaryotic cells - ampicillin gene. The U6 promoter of β-skeletal muscle actin gene (CMV-chicken-β-actin promoter) which can be extensively expressed can ensure the extensive expression of downstream genes.

### Embodiment 2 Construction of GGTA1/CMAH/β4GalNT2 triple knockout pigs by using a somatic cell cloning method

The GGTA1-CRISPR/Cas9 vector, CMAH-CRISPR/Cas9 vector and β4GalNT2-CRISPR/Cas9 vector constructed in Embodiment 1 were co-transfected into porcine fetal fibroblasts together with tdTomato plasmid. Single-cell clones were obtained by G418 screening and identified by sequencing to obtain GGTA1/CMAH/β4GalNT2 triple knockout porcine fetal fibroblasts. GGTAl/CMAH/β4GalNT2 triple knockout Landrace pigs were prepared by somatic cell nuclear transfer (SCNT). The genome of a newborn piglet was extracted, amplified by PCR primers, and ligated with a T vector for genotyping.

### Step I. Resuscitation of porcine primary fibroblasts

1. The cryopreserved porcine primary fibroblasts were taken out from liquid nitrogen and thawed in a water bath at 37°C;
2. The thawed cells were transferred into a 15 mL sterile centrifuge tube, into which was then added 3 mL cell medium and centrifuged at 1500 rpm for 5 min;
   Wherein, the formula of the complete cell medium was as below: 16% fetal bovine serum (Gibco) and 84% DMEM medium (Gibco), the percentages were percents by volume.
3. The supernatant was discarded, the cell precipitates were resuspended by adding 2 mL complete medium, and the resuspended cells were then plated in a 6 cm cell culture dish, into which was supplemented 2 mL complete medium, and placed in a constant temperature incubator at 37°C and 5% of CO₂ (percents by volume) for culture.
4. When the cells were cultured to confluent about 90% the bottom of the dish, they were digested with 0.05% (5 g/100 mL) of trypsin, and a complete medium was then added to terminate the digestion. The cell suspension was transferred into a 15 mL centrifuge tube, centrifuged at 1500 rpm for 5 min. The supernatant was discarded, and the cells were resuspended with 2 mL complete medium and counted. The total amount of cells was adjusted to 1.5×10⁶ for the next nucleofection.

### Step II. Co-transfection of porcine primary fibroblasts with the constructed GGTA1-PX330, CMAH-PX330, β4GalNT2-PX330 and tdTomato plasmid (Clontech, PT4069-5)

The nucleofection experiments were performed by using a mammalian fibroblast nucleofection kit (Lonza) and a Lonza Nucleofactor^{™} 2b nucleofection instrument.
1. Formulation of a nucleofection reaction liquid, the system was as follows:

| | |
|---|---|
| Basic solution for nucleofection | 82 µL |
| Supplement components | 8 µL |

2. The three constructed plasmids and the Tdtomato plasmid were added into the 100 µL nucleofection reaction liquid obtained in the previous step 1 at a mass ratio of 5:1 respectively and mixed evenly, being careful not to generate bubbles during the process;
3. The cell suspension prepared in step I was rinsed twice with Dulbecco's Phosphate Buffered Saline (DPBS, Gibco), digested at 37°C for 2 min. A DMEM complete medium containing fetal bovine serum at a volume percent of 10% was then used to terminate the digestion. After then, the suspension was centrigued at 1500 rpm for 5 min. The supernatant was discarded. The cells were resuspended with the nucleofection reaction liquid containing plasmids in the previous step 2, being careful to avoid the generation of bubbles during the process of resuspension;
4. The nucleofection system was added into the electroporation cuvettes contained in the kit carefully, being careful to prevent the bubbles. The electroporation cuvettes containing 100 µL PBS were firstly placed in the cuvette troughs of the Lonza nucleofector. After the program was adjusted by selecting U023 nucleofection procedures, the electroporation cuvettes containing cells were electroporated, and the liquid in the electroporation cuvettes was then immediately sucked out in a Clean Bench gently and transferred into 1 mL DMEM complete medium containing fetal bovine serum of 16% by volume, and mixed gently;
5. Preparing several culture dishes (10 cm) each containing 8 mL of complete medium. The cell suspension after nucleofection was pipetted and added into one culture dish containing the complete medium, and mixed evenly. The number of cells was observed under a microscope and counted such that the culture dish contained about 50 to 60 cells in a single field of view under the microscope. The remaining dishes were all added with the cell suspension following this final amount, mixed evenly and then placed in a constant temperature incubator at 37°C and 5% of CO₂ for culture.

### Step III. Screening on triple knockout cell lines

1. After the cells obtained in the step II were cultured for 24 h, the cell medium was replaced with a complete medium containing 1 mg/mL of G418, and placed in a constant temperature incubator at 37°C and 5% of CO₂ for culture. The cell medium was replaced every 2 to 3 days, during which the drug concentration of G418 was gradually reduced according to the growth profile of the cells. The final concentration of G418 was 0.3 mg/mL. After culture for about 10 to 14 days, monoclonal cell lines resistant to G418 would successively grew out in the culture dishes;
2. The cell lines were sorted by using cloning rings. The sorted monoclonal cell lines were inoculated in a 24-well plate plated with 0.3 mg/mL of G418 complete medium, and placed in a constant temperature incubator at 37°C and 5% of CO₂ for culture. The cell medium was replaced every 2 to 3 days;
3. When the cells were overgrown at the bottom of the wells in the 24-well plate, they were digested with trypsin and collected, wherein 4/5 of the cells were inoculated into a 12-well plate or a 6-well plate (according to the amount of cells) containing 0.3 mg/mL of G418 complete medium, and the remaining 1/5 of the cells were left in the 24-well plate to continue the culture;
4. When the bottom of the 12-well plate or the 6-well plate was covered with cells, the cells were digested with 0.05% (5 g/100 mL) of trypsin and collected, and cryopreserved with a cell freezing medium (90% fetal bovine serum + 10% DMSO, volume ratio);

### Step IV Gene identification of triple knockout cell lines

1. When the cells were overgrown at the bottom of the wells in the 24-well plate, they were digested with 0.05% (5 g/100 mL) of trypsin and collected. Then 25 ml NP-40 lysis buffer was added into the cells to lyse the cells and extract genomic DNA from the cells. The lysis procedures were: at 55°C for 60 min - at 95°C for 5 min - at 4°C. Upon the completion of the reaction, the genomic DNA was kept at -20°C;
2. Corresponding PCR primers were designed according to GGTA1/CMAH/β4GalNT2 gene target information. The PCR primer sequences were respectively:
GGTA1
   The forward primer was: 5'-CCTTAGTATCCTTCCCAACCCAGAC-3' (SEQ ID No: 13)
   The reverse primer was: 5'-GCTTTCTTTACGGTGTCAGTGAATCC-3' (SEQ ID No: 14)
   The PCR target product was 428 bp in length;
CMAH
   The forward primer was: 5'-CTTGGAGGTGATTTGAGTTGGG-3' (SEQ ID No: 15)
   The reverse primer was: 5'-CATTTTCTTCGGAGTTGAGGGC-3' (SEQ ID No: 16)
   The PCR target product was 485 bp in length;
β4GalNT2
   The forward primer was: 5'-CCCAAGGATCCTGCTGCC-3' (SEQ ID No: 17)
   The reverse primer was: 5'-CGCCGTGTAAAGAAACCTCC-3' (SEQ ID No: 18)
   The PCR target product was 399 bp in length;

3. The GGTA1/CMAH/β4GalNT2 target gene was amplied by PCR reaction. The PCR reaction system was as follows:

| | |
|---|---|
| Cell genomic DNA | 2 µL |
| GGTA1 Forward primer (10 pM) | 1 µL |
| GGTA1 reverse primer (10 pM) | 1 µL |
| 2X Taq enzyme premix | 25 µL |
| dd H₂O | 21 µL |
| Total | 50 µL |

The reaction conditions were as follows:

| | | | |
|---|---|---|---|
| Step 1 | 95°C | 5 min | 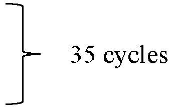 |
| Step2 | 95°C | 30 s | |
| | 64°C | 30 s | |
| | 72°C | 45 s | |
| Step3 | 72°C | 7 min | |
| Step4 | 4°C | ∞ | |

The amplification of CMAH target gene was performed the same as in the above steps; and the amplification of β4GalNT2 target gene was performed the same as in the above steps.
4. The PCR reaction products were subjected to agarose gel electrophoresis (1%, i.e., 1 g agarose gel being added into 100 mL electrophoresis buffer). At the end of electrophoresis, the target band was cut under ultraviolet light, and then recovered by a gel extraction kit (QIAGEN), and the concentration of the recovered PCR products was determined by using NanoDrop 200;
5. The recovered PCR products were ligated with T vectors by using a TAKARA pMD^{™}18-T Vector Cloning Kit. The T vector reaction system was as follows:

| | |
|---|---|
| pMD18-T vector | 1 µL |
| Gel recovered PCR products | 81.7 ng* |
| ddH₂O | Supplement the system to 10 µL |

| | |
|---|---|
| *Note: In the specification of the TAKARA pMD^{™}18-T Vector Cloning Kit, the amount of Insert DNA (here, gel recovered PCR products) was specified to be 0.1 to 0.3 pM, here 0.2 pM was selected. The amount was calculated as below: the amount of Insert DNA (ng) = the number of nmol × 660 × the number of bp of Insert DNA. | |

The reaction condition of T vector ligation was reaction at 16°C for 30 min;
6. The T vector ligation products obtained in the above step 5 were transformed with competent cells (TIANGEN). After the transformation, the competent cells were coated onto an Amp-resistant LB agar solid medium, and cultured in a constant temperature incubator at 37°C overnight;
10 to 15 monoclonal colonies were sorted from the medium cultured overnight and sent to a sequencing company for sequencing. The sequencing results were then compared with the target GGTA1/CMAH/β4GalNT2 information to determine whether the cell line was GGTA1/CMAH/β4GalNT2 gene knockout cell line;

A total of 27 monoclonal cell lines were sorted, wherein there was one biallelic knockout cell line in which three genes were knocked-out simultaneously, being numbered 50#. The genotype of the clone is shown in Table 1:

**Table 1 Gene identification of Landrace fibroblasts with the GGTA1/CMAH/β4GalNT2 gene knockout**

| | |
|---|---|
| GGTA | TTTTCCCAGGAGAAATAATGAATGTCAAAGGAAGAGTGGTTCTGTC WT |
| 50# | TTTTCCCAGGAGAAAATAATGAATGTtCAAAGGAAGAGTGGTTCTGTC +1 |
| CMAH | AGGTCCATGCAGGCGTGAGTAAGGTACGTGATCTGTTGGAAGACAGTWT |
| 50# | AGGTCCATGCAGGCGTGAGTAAaGGTACGTGATCTGTTGGAAGACAGT +1 |
| β4GalNT2 | GGGTAGTACTCACGAACACTCCGGAGCATGGTCATGAGCTTGTGGGGWT |
| 50# | GGGTAGT----------ACTCCGGAGCATGGTCATGAGCTTGTGGGG -10 |

It was found from the results that, the knockout efficiencies of knocking-out *GGTA1* (the nucleotide sequence of *GGTA1* fragment of WT in Table 1 is as shown in SEQ ID No: 19), CMAH (the nucleotide sequence of *CMAH* fragment of WT in Table 1 is as shown in SEQ ID No: 20), β4GalNT2 (the nucleotide sequence of *β4GalNT2* fragment of WT in Table 1 is as shown in SEQ ID No: 21) genes were 56%, 63% and 41%, respectively.

Since compared with GGTA1/CMAH double knockout, the binding to IgM, IgG of human is significantly reduced in GGTA1/CMAH/β4GalNT2 triple knockout, so triple knockout is necessary.

### Step V Somatic cell nuclear transfer

1. Sow ovarians at an age of six months or above were purchased from a slaughter house. The immature oocytes in the follicle were extracted artificially. The oocytes of good quality were sorted under a microscope and cultured in a constant temperature incubator at 38.5°C and 5% of CO₂ for 42 to 44 h until the maturation of oocytes;
2. The mature oocytes in the above step (1) were denucleated with a micromanipulation system. The GGTA1/CMAH/β4GalNT2 knockout monoclonal cell lines obtained in Step IV were then resuscitated. The GGTA1/CMAH/β4GalNT2 knockout cells were injected into the denucleated oocytes as nuclear donors, wherein one GGTA1/CMAH/β4GalNT2 knockout cell was injected into each denucleated oocyte.
3. The injected cells were then be used to activate the reconstructed embryos after nuclear transfer by using an electrofusion technology. The embryos were cultured in an incubator at 38.5°C for 5 days until they developed into morula;
4. The well-developed embryos were implanted into the womb of a surrogate sow. The surrogate sow was taken care carefully. One month after the implantation, the pregnancy profile in the recipient pig was detected with B ultrasound. The surrogate sow was monitored in real time until it labored.

### Step VI. Genotyping of triple knockout Landrace

1. After the GGTA1/CMAH/β4GalNT2 gene knockout piglets were born, the ear tissue was cut from the piglets, and then genomic DNA was extracted from the piglets by using a blood/cell/tissue genomic DNA isolation kit (TIANGEN);
2. The piglet genomic DNA obtained in the above step 1 was subjected to PCR reaction, the conditions for which were the same as in Step IV, 3. The PCR reaction products were then sent to a sequencing company for sequencing. The sequencing results were compared the GGTA1/CMAH/β4GalNT2 gene target sequence.

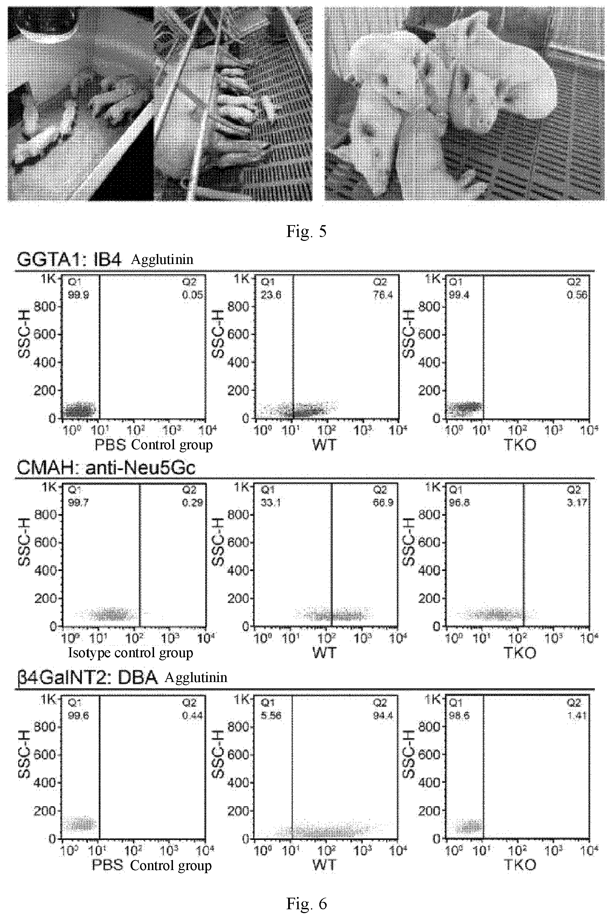

A total of 8 GGTA1/CMAH/β4GalNT2 knockout pigs (TKO) were born, being numbered from 1 to 8 (as shown in Fig. 5). The 8 born boars were consistent with the results of cell genotyping.

Following the steps in Embodiment 2, the GGTA1-CRISPR/Cas9 vector constructed in Embodiment 1 was used alone to get GGTA1 single gene knockout (GGTA1-KO) pigs.

### Embodiment 3 Properties of GGTA1/CMAH/β4GalNT2 knockout pigs

### 3.1. GGTA1/CMAH/β4GalNT2 in the GGTA1/CMAH/β4GalNT2 knockout pigs was determined to be knocked out

After the GGTA1/CMAH/β4GalNT2 knockout pigs prepared in Embodiment 2 were weaned, blood was drawn and peripheral blood mononuclear cells (PBMC) were isolated, and the gene knockout profile of the piglets was determined by flow cytometer.

PBMC were isolated as follows: To 100 µL anticoagulant blood was added 3 times volume of red blood cell lysis buffer (BD, diluted with deionized water by 10 times), and the lysis was performed at room temperature for 5 min to 10 min. After centrifugation, the supernatant was discarded. The remainings were rinsed with a pre-cooled washing liquid 0.1% FBS (the solvent was PBS, 0.1% means 0.1 g FBS/100 mL PBS)(promote cell sedimentation), and centrifuged to obtain PBMC precipitates.

The commercialized human serum was inactivated in a water bath kettle at 56°C for 30 min and then used to incubate the obtained PBMC on ice for 2 h, which were then centrifuged at 5000 rpm for 5 min, washed with PBS for three times, blocked with goat serum with a volume ratio of 10% at 4°C for 30 min, and washed with PBS for additional three times. After incubation with antibodies specifically binding to GGTA1, CMAH and β4GalNT2, the antibodies were washed away with PBS, resuspended and the mean fluorescence intensity was determined on a machine.

The results were shown in Fig. 6, which, from top to bottom, showed the expression profiles of GGTA1, CMAH and β4GalNT2 in sequence. Wherein, PBS control group was the blank control, the isotype control group was chick IgY, WT was wild-type pig. The results showed that, the three antigens (α-1,3-galactosyl transferase (GGTA1), CMP-N-acetylneuraminic acid hydroxylase (CMAH) and β-1,4-N-acetylgalactosaminyl transferase 2 (β4GalNT2)) were not expressed in TKO pigs. In other words, *GGTA1* gene, *CMAH* gene and *β4GalNT2* gene in TKO have all been knocked out successfully.

### 3.2. Binding level of peripheral blood mononuclear cells (PBMC) to immunoglobulin in human serum

PBMC were separated from GGTA1/CMAH/β4GalNT2 knockout pigs (TKO), GGTA1-KO pigs prepared in Embodiment 2, as well as human and wild-type pigs following the process as described in 3.1.

The commercialized human serum was inactivated in a water bath kettle at 56°C for 30 min and then used to incubate the obtained PBMC on ice for 2 h, which were then centrifuged at 5000 rpm for 5 min, washed with PBS for three times, blocked with goat serum with a volume ratio of 10% at 4°C for 30 min, and washed with PBS for additional three times. After incubation with human specific immunoglobulin antibodies (i.e., anti-human IgM antibody and anti-human IgG antibody), the antibodies were washed away with PBS, resuspended and the mean fluorescence intensity was determined on a machine.

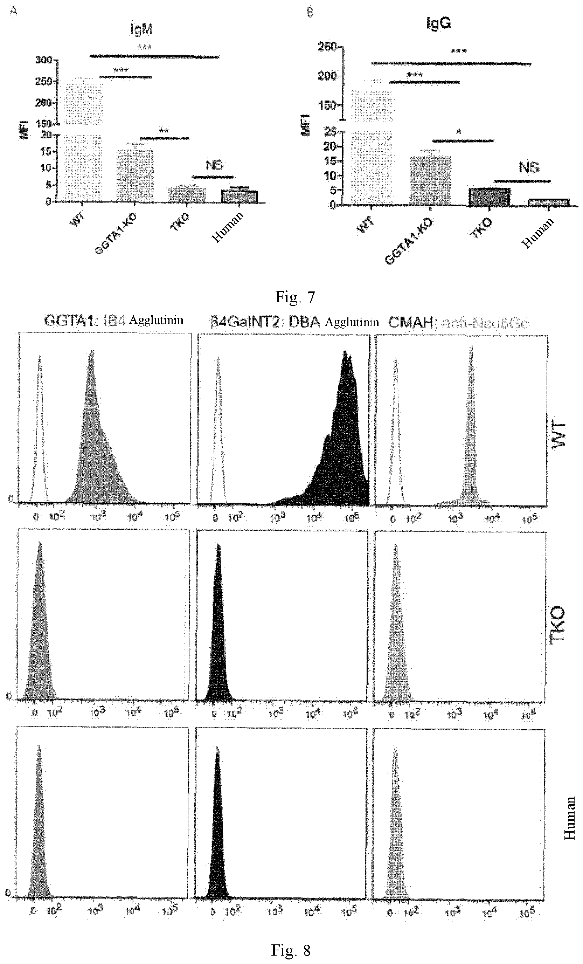

The results were shown in Figs. 7A-7B, which showed the level of binding to immunoglobulins IgM and IgG in human serum in sequence. The results showed that, compared with wild-type pigs, the binding level of PBMC of TKO to human immunoglobulins IgM and IgG was greatly reduced, with little difference from the level of binding to human PBMC in normal circumstances. However, although GGTA1-KO pigs are a little superior to wild-type pigs, the level of binding to human immunoglobulins IgM and IgG was still significantly different from that to human PBMC. It can be seen that PBMCs of TKO were capable of overcoming human hyperacute rejection.

### Embodiment 4 Characteristics of RBC of TKO

### (1) Separation of red blood cells (RBCs)

The GGTA1/CMAH/β4GalNT2 knockout pigs (TKO) prepared in Embodiment 2 were immobilized, from the anterior vena cava of which 5 mL blood was drawn with a sterile syringe, and placed in an anticoagulation tube and preserved at 4°C for one week. 2 mL of the above anticoagulant blood was taken and added into a 15 mL centrifuge tube, and then 2 mL PBS solution was added for dilution and mixed evenly. The diluted blood was slowly added into a 15 mL centrifuge tube containing 3 mL Ficoll-paque separation liquid (GE Company), at which there were two layers, wherein the upper layer was blood, and the lower layer was Ficoll-paque separation liquid. After centrifugation at 19°C and at 400 g for 40 min, the liquid phase was divided into four layers, which were successively, from top to bottom, a plasma layer, a monocyte layer, a Ficoll-paque layer and a red blood cell layer. The supernatant was discarded, and the red blood cells were remained and resuspended with 7 mL PBS solution and mixed evenly. After centrifugation at 19°C and at 400 g for 10 min, the supernatant was discarded, and 5 mL PBS solution was added for resuspension and mixed evenly. After centrifugation at 19°C and at 400 g for 10 min, the supernatant was discarded, and 2 mL PBS was added for resuspension, ready for use.

Following this process, human RBCs and RBCs of wild-type pigs (WT) can be obtained respectively.

### (2) Incubation with IB4 agglutinin or DBA

IB4 agglutinin interacted with carbohydrates ligated to α galactose generated from the expression products of *GGTA1*; and DBA agglutinin interacted with the structures of carbohydrates generated from the expression products of *β4GalNT2.*

1×10⁵ red blood cells prepared in step (1) were placed in a 1.5 mL EP tube and centrifuged at 3000 rpm for 5 min, discarding the supernatant. The cell precipitates were resuspended with 200 µL of IB4 agglutinin (purchased from Invitrogen) or DBA agglutinin (purchased from Invitrogen) dilution (at a dilution ratio of 1:1000) diluted with PBS, and incubated at 4°C in dark for 1 h. The samples which have not been incubated with agglutinin were used as blank control. They were washed twice with a PBS solution, the centrifuged precipitates were resuspended with 200 µL of PBS solution, detected with BD FACSCalibur flow cytometry, and analyzed using FlowJo 10.0 software, with the results being shown in Fig. 8.

Columns 1 and 2 in Fig. 8 successively showed the results after incubation with IB4 agglutinin and DBA agglutinin respectively. Wherein, WT means wild-type pigs. It was indicated from the results in Fig. 8 that, unlike WT, the antigen flow results of RBC of TKO and human (RBC of human type O) against IB4 agglutinin and DBA agglutinin were all negative.

### (3) Incubation with Neu5Gc antibody

*CMAH* gene was capable of synthesizing saccharide molecule Neu5Gc.

1×10⁵ red blood cells prepared in step (1) were placed in a 1.5 mL EP tube and centrifuged at 3000 rpm for 5 min, discarding the supernatant. The cells were resuspended with 200 µL of 0.5% diluted blocking liquid (free from mammal serum) and incubated at 4°C in dark for 30 min. They were washed twice with a PBS solution, the centrifuged precipitates were then resuspended with 200 µL of Neu5Gc antibody (Purified anti-Neu5Gc Antibody (biolegend, 146903)) dilution (at a dilution ratio of 1: 1000) diluted with a PBS solution, and incubated at 4°C for 1 h. The samples which have not been incubated with antibodies were used as blank control. They were washed twice with a PBS solution, and the cell precipitates were then resuspended with 200 µL of goat-anti-chick IgY antibody (invitrogen, A11039) dilution (at a dilution ratio of 1:1000) diluted with a PBS solution, incubated at 4°C in dark for 1 h, and centrifuged at 3000 rpm for 5 min, discarding the supernatant. They were washed twice with a PBS solution, the centrifuged precipitates were then resuspended with 200 µL of PBS solution, detected with BD FACSCalibur flow cytometry, and analyzed using FlowJo 10.0 software, with the results being shown in Fig. 8.

Column 3 in Fig. 8 showed the results after incubation with Neu5Gc antibody. Wherein, WT means wild-type pigs. It was indicated from the results in Fig. 8 that, unlike WT, the antigen flow results of RBC of TKO and human (RBC of human type O) against Neu5Gc antibody were all negative.

### (4) Human IgG/IgM binding experiment

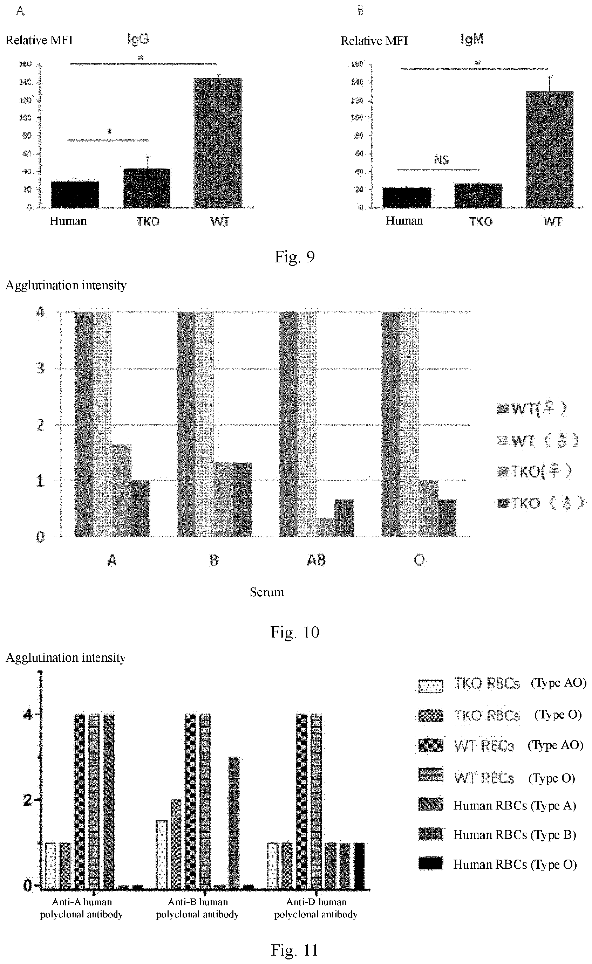

Human type AB serum was inactivated in advance by incubation at 56°C for 30 min. 1×10⁵ red blood cells prepared in step (1) were placed in a 1.5 mL EP tube and centrifuged at 3000 rpm for 5 min, discarding the supernatant. The cell precipitates were resuspended with 200 µL of 15% (v/v) human AB serum dilution diluted with a PBS solution and incubated at 4°C for 1 h. The samples which have not been incubated with human AB serum were used as blank control. They were washed twice with a PBS solution, and the cells were then resuspended with 200 µL of 10% (v/v) ready-to-use normal goat serum and incubated at 4°C for 30 min. They were washed twice with a PBS solution, and the cell precipitates were then resuspended with 200 µL of goat-anti-human IgG or IgM antibody (anti-human IgM (invitrogen, A18842); anti-human IgG (invitrogen, A18830) dilution (at a dilution ratio of 1:1000) diluted with a PBS solution, incubated at 4°C in dark for 1 h, and centrifuged at 3000 rpm for min, discarding the supernatant. They were washed twice with a PBS solution, the centrifuged precipitates were then resuspended with 200 µL of PBS, detected with BD FACSCalibur flow cytometry, and analyzed using FlowJo 10.0 software, with the results being shown in Figs. 9A-9B.

The results in Figs. 9A-9B showed that WT means wild-type pigs. It was indicated from the results in Figs. 9A-9B that, the capabilities of human red blood cells and red blood cells of TKO to bind to human IgG and IgM were all significantly lower than that of red blood cells of wild-type pigs. It can be seen that RBCs of TKO are capable of overcoming human hyperacute rejection.

### Embodiment 5 Red blood cell agglutination test

### 5.1 Agglutination test

See Embodiment 4 for the acquisition process of RBC.

The collected porcine blood, after being washed for three times, was formulated to a 3% red blood cell suspension. 50 µL of 3% (v/v) RBCs (WT pigs and TKO pigs) suspension and 100 µL of normal human (types A, B, AB and O) serum were respectively added into glass test tubes, incubated at 37°C for 30min, centrifuged at 1500 g for 30 s, discarding the supernatant. They were washed with normal saline for 3 times, into which were respectively added 25 µL of anti-human IgG antibodies (Shanghai Blood Biomedicine Co. LTD), centrifuged at 1000 g for 15 s at ambient temperature, and shaked gently. After then, the agglutination degrees were observed, with the results being shown in Fig. 10.

It was indicated from the results in Fig. 10 that, regardless of gender, the agglutination intensities between RBCs of TKO pigs and human type A, B, AB and O sera were significantly lower than the agglutination intensities between RBCs of wild-type pig and human type A, B, AB and O sera. The agglutination degrees were determined following the vertical coordinates in Fig. 10: 0: No agglutination or hemolysis; ±: turbid background, small scattered incompact agglutination blocks, after shaking, the agglutination blocks became invisible; 1+: turbid background, small scattered incompact agglutination blocks, after shaking, the agglutination blocks were still visible; 2+: incompact agglutination blocks, clear background, after shaking, the background became turbid; 3+: several compact agglutination blocks, clear background; 4+: one compact agglutination block. With the increase of the number, the agglutination degree increased continuously.

Each 2 drops of anti-A human polyclonal antibody, anti-B human polyclonal antibody and anti-D human polyclonal antibody (all purchased from The Institute of Blood Transfusion, Chinese Academy of Medical Sciences) were added into 1 drop of 3% RBCs (WT pigs, TKO pigs and human) suspension to be detected after being washed for three times. They were added into a test tube together, mixed evenly, and centrifuged at 1000 g for 15 seconds. The agglutination degrees were observed by naked eyes, with the results being shown in Figs. 11-12.

It was indicated from the results in Fig. 11 that compared with red blood cells of wild-type pigs, the agglutination degree of red blood cells of TKO pigs to human serum was significantly weakened.

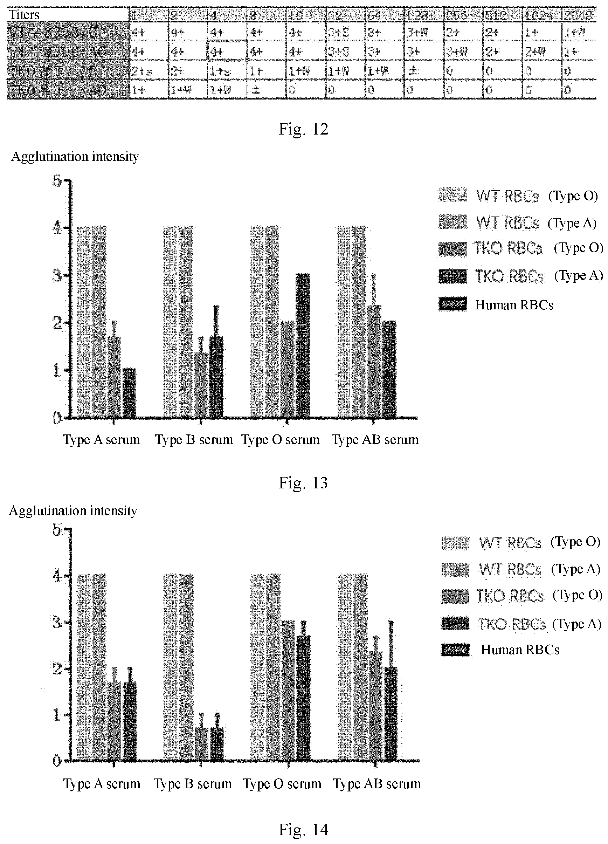

Fig. 12 shows the agglutination titers of red blood cells of WT pigs and TKO pigs to human AB serum respectively. The maximum dilution of RBC at which obvious agglutination phenomenon appeared was used as the agglutination titer. In Fig. 12, the agglutination degrees were determined as follows: 0: No agglutination or hemolysis; ±: turbid background, small scattered incompact agglutination blocks, after shaking, the agglutination blocks became invisible; 1+: turbid background, small scattered incompact agglutination blocks, after shaking, the agglutination blocks were still visible; 2+: incompact agglutination blocks, clear background, after shaking, the background became turbid; 3+: several compact agglutination blocks, clear background; 4+: one compact agglutination block. With the increase of the number, the agglutination degree increased continuously. +S means strong, i.e., strengthened; +W means weak, i.e., weakened. 3906 (type A) and 3353 (type O) blood samples were taken from WT pigs, 0 (type A) and 3 (type O) blood samples were taken from TKO pigs.

### 5.2 Determination of red blood cell agglutination by saline method

See Embodiment 4 for the acquisition process of RBCs (WT pigs, TKO pigs and human). These RBCs were used as donor red blood cells.

Human type A, type B, type AB, and type O sera (all taken from healthy blood donors) were used as recipient sera.

Two tiny test tubes were taken and marked as the main tube and the self control tube respectively. The main tube was added with 2 drops of recipient serum and 1 drop of donor red blood cell suspension; the self control tube was added with 2 drops of recipient serum and 1 drop of recipient red blood cell suspension. They were mixed evenly by shaking and centrifuged at 1000 g for 15 seconds. The agglutination degrees were observed by naked eyes, with the results being shown in Fig. 13.

It was indicated from the results in Fig. 13 that during the determination of red blood cell agglutination caused by an IgM antibody against a blood group antigen, the agglutinations of red blood cells of TKO pigs in various blood group sera of human were significantly reduced compared to those of red blood cells of wild-type pigs.

### 5.3 Determination of red blood cell agglutination by indirect antihuman globulin method

See Embodiment 4 for the acquisition process of RBCs (WT pigs, TKO pigs and human). These RBCs were used as donor red blood cells.

Human type A, type B, type AB, and type O sera (all taken from healthy blood donors) were used as recipient sera.

The loading of the main tube and the self control tube was achieved following the operational steps of saline method as described in Embodiment 5.2. The mixture was mixed evenly and incubated at 37°C for 30 minutes. Red blood cells were washed for three times, and the tubes were dried by patting after the last washing. Each tube was added with 1 drop of antihuman IgG antibody (purchased from Shanghai Blood Biomedicine Co. LTD), mixed, and centrifuged at 1000 g for 15 seconds. The results were observed and shown in Fig. 14.

It was indicated from the results in Fig. 14 that during the determination of red blood cell agglutination caused by an IgG antibody against a blood group antigen, the agglutinations of red blood cells of TKO pigs in various blood group sera of human were significantly reduced compared to those of red blood cells of wild-type pigs.

### Embodiment 6 Human monocyte to macrophage differentiation associated protein (MMA) test

### Materials and Methods

### 1. Blood specimens

Human blood samples were taken from blood donors, who meet the National standards for blood donors' health examination. 5 mL of whole blood was draw from each person, and preserved at 4°C, ready for use. 5497 (type A), 5119 (type O) blood samples were taken from WT pigs, 0# (type A), 3# (type O) blood samples were taken from TKO pigs.

### 2. Instruments and reagents

Lymphocyte separation liquid (AS1114545, Axis-Shield, Norway), RPMI 1640 basic medium (gibco, US), fetal bovine serum (FBS, 0500, Sciencell, US), Wright-Giemsa Stain (DN0007, Leagene Biotech. Co., Ltd), Methanol (Sinopharm). Chamber system (154534PK, Thermo Fisher, US), Upright Microscope (BX53, Olympus, Japan).

### 3. Separation and cultivation of human peripheral blood mononuclear cells (PBMCs)

5 mL of the whole blood was transferred into a 50 mL centrifuge tube, into which was added an equal amount of PBS for dilution and mixed evenly. Into a 50 mL centrifuge tube was added 10 mL of the lymphocyte separation liquid, and the diluted blood was added gently to the top of the lymphocyte separation liquid in the centrifuge tube, and centrifuged at 2200 rpm at room temperature for 20 min, during which the centrifugal speed rose and fell slowly. After centrifugation, the cell layer at which PBMC were located was white. This layer of cells were pipetted into another 50 mL centrifuge tube, which were washed twice by adding PBS, resuspended in RPMI 1640 +10% FBS medium, and cultivated in the chamber system (500 µL/well, 700000 cells), and incubated in an incubator at 37°C and 5% of CO₂ for 1 hour to make them adhere to the wall.

### 4. Co-incubation treatment of porcine red blood cells (pRBCs) and sera

Porcine red blood cells pRBC (WT pigs and TKO pigs) were drawn, washed twice with PBS, and counted. Sera were drawn from human whole blood. 1.4×10⁸ red blood cells were mixed with 200 µL of sera evenly, incubated in an incubator at 37°C and 5% of CO₂ for 1 hour. A positive control group (Incubation of human-derived anti-D antibody and human red blood cells) and a negative control group (Incubation of type AB serum and human type O red blood cells) were set. They were washed with PBS for 3 times, resuspended in 500 µL RPMI 1640 +10% FBS medium.

### 5. MMA phagocytosis test

The adherent PBMCs were sucked out of the medium, then 500 µL of pRBCs which have been reacted with the serum were added, incubated in an incubator at 37°C and 5% of CO₂ for 2 hours, rinsed with PBS twice, immobilized with methanol at room temperature for 45 s, and stained with Wright-Giemsa Stain at room temperature for 1 minute; an equal amount of phosphate dilution was added and left at room temperature for 5 min. The stain was then flushed away with water. It was dried and then photographed.

The mean phagocytic index was determined as follows: observation using a microscope, photographing, and counting more than 600 cells. The mean phagocytic index = Number of adherent or phagocytic red blood cells/Total number of cells × 100%.

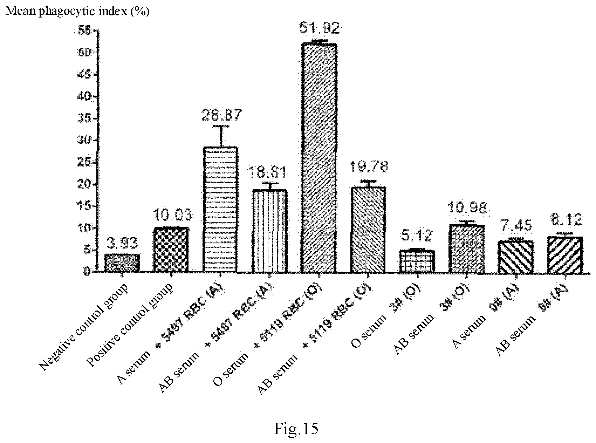

The results of mean phagocytic index can be found in Fig. 15. It was indicated from the results in Fig. 15 that the likelihood of a hemolytic transfusion reaction occurring after the red blood cells of TKO pigs are introduced into a human body was far lower than that of red blood cells of wild-type pigs.

## Claims

1. A method of preparing a blood product, comprising preparing a gene knockout pig and isolating a blood product from said gene knockout pig;
wherein said gene knockout pig is prepared through somatic cell nuclear transfer by using a CRISPR/Cas9 vector combination; the exon 3 of said GGTA1 gene, the exon 6 of said CMAH gene and the exon 8 of said β4GalNT2 gene serve as the parts targeted by CRISPR/Cas9;
wherein the CRISPR/Cas9 vector combination comprises a GGTA1-CRISPR/Cas9 vector, a CMAH-CRISPR/Cas9 vector and a β4GalNT2-CRISPR/Cas9 vector, said GGTA1-CRISPR/Cas9 vector contains the sgRNA nucleotide sequence specifically targeting the GGTA1 gene of SEQ ID No: 1, said CMAH-CRISPR/Cas9 vector contains the sgRNA nucleotide sequence specifically targeting the CMAH gene of SEQ ID No: 2, and said β4GalNT2-CRISPR/Cas9 vector contains the sgRNA nucleotide sequence specifically targeting the β4GalNT2 gene of SEQ ID No: 3.

2. The method according to claim 1, wherein said GGTA1-CRISPR/Cas9 vector comprises the nucleotide sequence of SEQ ID No: 4, said CMAH-CRISPR/Cas9 vector comprises the nucleotide sequence of SEQ ID No: 5, and said β4GalNT2-CRISPR/Cas9 vector comprises the nucleotide sequence of SEQ ID No: 6.

3. The method according to any one of claims 1-2, wherein the blood product comprises red blood cells and/or peripheral blood mononuclear cells (PBMC) of said gene knockout pig; optionally, said red blood cells have a reduced αGal antigen level, a reduced Neu5Gc antigen level and a reduced Sd^{a}-like antigen level; and/or said PBMC have a reduced αGal antigen level, a reduced Neu5Gc antigen level and a reduced Sd^{a}-like antigen level.

4. The method according to claim 3, wherein the binding level of the red blood cells of said gene knockout pig to human immunoglobulin is reduced compared to red blood cells derived from a wild-type pig; and/or the binding level of the PBMC of said gene knockout pig to human immunoglobulin is reduced compared to PBMC derived from a wild-type pig; optionally, said human immunoglobulin comprises human IgGand/or human IgM.

5. The method according to any one of claims 3-4, wherein the red blood cells of said gene knockout pig have a comparable level of binding to human immunoglobulin compared to human-derived red blood cells; and/or the PBMC of said gene knockout pig have a comparable level of binding to human immunoglobulin compared to human-derived PBMC; optionally, said human immunoglobulin comprises human IgGand/or human IgM.

6. The method according to any one of claims 3-5, wherein the agglutination reaction of the red blood cells of said gene knockout pig in human serum is reduced compared to red blood cells derived from a wild-type pig; optionally said agglutination reaction is caused by an IgM antibody against a blood group antigen and/or an IgG antibody against a blood group antigen.

7. The method according to any one of claims 3-6, wherein the likelihood of a hemolytic transfusion reaction occurring after the red blood cells of said gene knockout pig are introduced into a human body is reduced compared to red blood cells derived from a wild-type pig.

## Patentansprüche

1. Verfahren zum Herstellen eines Blutprodukts, das Herstellen eines Gen-Knockout-Schweins und Isolieren eines Blutprodukts aus diesem Gen-Knockout-Schwein umfasst;
wobei das Gen-Knockout-Schwein durch somatischen Zellkerntransfer unter Verwendung einer CRISPR/Cas9-Vektorkombination hergestellt wird; das Exon 3 des GGTA1-Gens, das Exon 6 des CMAH-Gens und das Exon 8 des β4GalNT2-Gens als die Teile dienen, auf die das CRISPR/Cas9 abzielt;
wobei die CRISPR/Cas9-Vektorkombination einen GGTA1-CRISPR/Cas9-Vektor, einen CMAH-CRISPR/Cas9-Vektor und einen β4GaINT2-CRISPR/Cas9-Vektor umfasst, wobei der GGTA1-CRISPR/Cas9-Vektor die sgRNA-Nukleotidsequenz enthält, die spezifisch auf das GGTA1-Gen von SEQ ID Nr. 1 abzielt, der CMAH-CRISPR/Cas9-Vektor die sgRNA-Nukleotidsequenz enthält, die spezifisch auf das CMAH-Gen von SEQ ID Nr. 2 abzielt, und der β4GaINT2-CRISPR/Cas9-Vektor die sgRNA-Nukleotidsequenz enthält, die spezifisch auf das β4GaINT2-Gen von SEQ ID Nr. 3 abzielt.

2. Verfahren nach Anspruch 1, wobei der GGTA1-CRISPR/Cas9-Vektor die Nukleotidsequenz von SEQ ID Nr. 4 umfasst, der CMAH-CRISPR/Cas9-Vektor die Nukleotidsequenz von SEQ ID Nr. 5 umfasst, und der β4GaINT2-CRISPR/Cas9-Vektor die Nukleotidsequenz von SEQ ID Nr. 6 umfasst.

3. Verfahren nach einem der Ansprüche 1-2, wobei das Blutprodukt rote Blutzellen und/oder periphere mononukleäre Blutzellen (PBMC) des Gen-Knockout-Schweins umfasst; gegebenenfalls die roten Blutzellen einen reduzierten aGal-Antigen-Spiegel, einen reduzierten NeuSGc-Antigen-Spiegel und einen reduzierten Sd^{a}-ähnlichen Antigen-Spiegel aufweisen; und/oder die PBMC einen reduzierten aGal-Antigen-Spiegel, einen reduzierten NeuSGc-Antigen-Spiegel und einen reduzierten Sd^{a}-ähnlichen Antigen-Spiegel aufweisen.

4. Verfahren nach Anspruch 3, wobei der Bindungsgrad der roten Blutzellen des Gen-Knockout-Schweins an humanes Immunglobulin im Vergleich zu roten Blutzellen, die von einem Wildtyp-Schwein stammen, reduziert ist; und/oder der Bindungsgrad der PBMC des Gen-Knockout-Schweins an humanes Immunglobulin im Vergleich zu PBMC, die von einem Wildtyp-Schwein stammen, reduziert ist; gegebenenfalls das humane Immunglobulin humanes IgG und/oder humanes IgM umfasst.

5. Verfahren nach einem der Ansprüche 3-4, wobei die roten Blutkörperchen des Gen-Knockout-Schweins einen vergleichbaren Grad an Bindung an menschliches Immunglobulin aufweisen im Vergleich zu von Menschen stammenden roten Blutkörperchen; und/oder die PBMC des Gen-Knockout-Schweins einen vergleichbaren Grad an Bindung an menschliches Immunglobulin aufweisen im Vergleich zu von Menschen stammender PBMC; gegebenenfalls das menschliche Immunglobulin menschliches IgG und/oder menschliches IgM umfasst.

6. Verfahren nach einem der Ansprüche 3-5, wobei die Agglutinationsreaktion der roten Blutkörperchen des Gen-Knockout-Schweins in menschlichem Serum im Vergleich zu roten Blutkörperchen, die von einem Wildtyp-Schwein stammen, reduziert ist; gegebenenfalls die Agglutinationsreaktion durch einen IgM-Antikörper gegen ein Blutgruppenantigen und/oder einen IgG-Antikörper gegen ein Blutgruppenantigen verursacht wird.

7. Verfahren nach einem der Ansprüche 3-6, wobei die Wahrscheinlichkeit einer hämolytischen Transfusionsreaktion, die auftritt, nachdem die roten Blutkörperchen des Gen-Knockout-Schweins in einen menschlichen Körper eingebracht wurden, im Vergleich zu roten Blutkörperchen, die von einem Wildtyp-Schwein stammen, reduziert ist.

## Revendications

1. Procédé de préparation d'un produit sanguin, comprenant la préparation d'un porc à inactivation de gène et l'isolement d'un produit sanguin dudit porc à inactivation de gène ;
dans lequel ledit porc à inactivation de gène est préparé par transfert nucléaire de cellules somatiques en utilisant une combinaison de vecteurs CRISPR/Cas9 ; l'exon 3 dudit gène GGTA1, l'exon 6 dudit gène CMAH et l'exon 8 dudit gène β4GalNT2 servent de parties ciblées par CRISPR/Cas9 ;
dans lequel la combinaison de vecteurs CRISPR/Cas9 comprend un vecteur GGTA1-CRISPR/Cas9, un vecteur CMAH-CRISPR/Cas9 et un vecteur β4GalNT2-CRISPR/Cas9, ledit vecteur GGTA1-CRISPR/Cas9 contient la séquence nucléotidique d'ARN guide ciblant précisément le gène GGTA1 de SEQ ID No : 1, ledit vecteur CMAH-CRISPR/Cas9 contient la séquence nucléotidique d'ARN guide ciblant précisément le gène CMAH de SEQ ID No : 2, et ledit vecteur β4GalNT2-CRISPR/Cas9 contient la séquence nucléotidique d'ARN guide ciblant précisément le gène β4GalNT2 de SEQ ID No : 3.

2. Procédé selon la revendication 1, dans lequel ledit vecteur GGTA1-CRISPR/Cas9 comprend la séquence nucléotidique de SEQ ID No : 4, ledit vecteur CMAH-CRISPR/Cas9 comprend la séquence nucléotidique de SEQ ID No : 5, et ledit vecteur β4GalNT2-CRISPR/Cas9 comprend la séquence nucléotidique de SEQ ID No : 6.

3. Procédé selon l'une quelconque des revendications 1-2, dans lequel le produit sanguin comprend des globules rouges et/ou des cellules mononucléées du sang périphérique (PBMC) dudit porc à inactivation de gène ; facultativement, lesdits globules rouges présentent un taux d'antigène αGal réduit, un taux d'antigène Neu5Gc réduit et un taux d'antigène de type Sd^{a} réduit ; et/ou lesdites PBMC présentent un niveau d'antigène αGal réduit, un niveau d'antigène Neu5Gc réduit et un niveau d'antigène de type Sd^{a} réduit.

4. Procédé selon la revendication 3, dans lequel le niveau de liaison des globules rouges dudit porc à inactivation de gène à l'immunoglobuline humaine est réduit par rapport aux globules rouges dérivés d'un porc de type sauvage ; et/ou le niveau de liaison des PBMC dudit porc à inactivation de gène à l'immunoglobuline humaine est réduit par rapport aux PBMC dérivées d'un porc de type sauvage ; facultativement, ladite immunoglobuline humaine comprend une IgG humaine et/ou une IgM humaine.

5. Procédé selon l'une quelconque des revendications 3-4, dans lequel les globules rouges dudit porc à inactivation de gène présentent un niveau de liaison à l'immunoglobuline humaine comparable à celui des globules rouges d'origine humaine ; et/ou les PBMC dudit porc à inactivation de gène présentent un niveau de liaison à l'immunoglobuline humaine comparable à celui des PBMC d'origine humaine ; facultativement, ladite immunoglobuline humaine comprend une IgG humaine et/ou une IgM humaine.

6. Procédé selon l'une quelconque des revendications 3-5, dans lequel la réaction d'agglutination des globules rouges dudit porc à inactivation de gène dans le sérum humain est réduite par rapport aux globules rouges dérivés d'un porc de type sauvage ; facultativement, ladite réaction d'agglutination est provoquée par un anticorps IgM contre un antigène de groupe sanguin et/ou un anticorps IgG contre un antigène de groupe sanguin.

7. Procédé selon l'une quelconque des revendications 3-6, dans lequel la probabilité qu'une maladie hémolitique par incompatibilité sanguine se produise après que les globules rouges dudit porc à inactivation de gène sont introduits dans un corps humain est réduite par rapport aux globules rouges dérivés d'un porc de type sauvage.
